# EUROPEAN PATENT SPECIFICATION

(11) **EP 0 384 731 B1**
(45) Date of publication and mention of the grant of the patent: **18.12.1996**
(21) Application number: 90301862.0
(22) Date of filing: 21.02.1990
(51) Int. Cl.: C07K 7/00, C07K 4/12, A61K 38/04, A61K 38/03, C07K 1/14, G01N 33/50

(54) **Osteogenic growth polypeptides identified from regenerating bone marrow**
Osteogenische Wachstumfaktoren, die aus sich regenerierendem Knochenmark identifiziert werden
Facteurs de croissance ostéogéniques identifiés à partir de moelle osseuse régénératrice

(30) Priority: 23.02.1989 US 314602
(43) Date of publication of application: 29.08.1990
(73) Proprietor: YISSUM RESEARCH DEVELOPMENT COMPANY OF THE HEBREW UNIVERSITY OF JERUSALEM, Jerusalem 91042 (IL)
(72) Inventor: Rodan, Gideon A., Bryn Mawr, PA 19010 (US); Sardana, Mohinder K., Lansdale, PA 19446 (US); Jacobs, John W., Doylestown, PA 18901 (US); Gazit, Dan, Jerusalem, 92546 (IL); Bab, Itai A., Jerusalem, 92546 (IL)
(74) Representative: Goldin, Douglas Michael

(56) References cited:
- EP-A- 0 241 578
- EP-A- 0 349 048
- US-A- 4 434 094
- US-A- 4 455 256
- CHEMICAL ABSTRACTS, vol. 74, no. 1, 4 January 1971, Columbus, Ohio, USA, page 169; Sautiere et al
- CHEMICAL ABSTRACTS, vol. 76, no. 7, 14 February 1972, Columbus, Ohio, USA; Sautiere P. et al: "Primary structure of the glycine- and arginine- rich histone isolated from rat chloroleukemic tumor", page 98

## Description

### BACKGROUND OF THE INVENTION

It is well known that after marrow ablation there is an osteogenic phase where trabeculae of primary bone replace the blood clot and fill the marrow space. The trabeculae are then subjected to osteoclastic resorption that precedes the appearance of regenerated normal marrow. Not only is there osteogenic reaction locally in the marrow cavity, there is stimulation of bone formation in cortical osteons and enhancement of osteo- and chondrogenesis in distant skeletal sites. Observations in mandibular condyles during the osteogenic phase of postablation healing of tibial marrow suggested that the enhanced osteogenesis resulted from an increase in both the number and activity of osteoblasts. It has been proposed that a factor or factors are produced locally by the regenerating marrow that mediate the peripheral osteogenic response after their release into the blood circulation. Bab I. et al., (1988) Endocrinology 123:345; Bab. I. et al., (1985) Calcif Tissue Int 37:551.

US-A-4,434,094 describes a process for partially purifying an osteogenic factor from demineralized bone particles by fractionation by anion exchange chromatography using DEAE cellulose at pH7. The partially purified osteogenic factor was recovered as a ≦ 30,000 dalton protein isolate.

The present invention establishes that regenerating bone marrow produces growth factor activity with an effect on osteogenic cells. Additionally, the present invention provides a novel osteogenic growth polypeptide, identified from regenerating bone marrow, which (i) has a stimulatory effect on osteoblastic cells, and (ii) promotes in vivo bone formation.

The novel osteogenic growth polypeptide of the present invention has sequence homology with histone H4, a 102 amino acid protein, and with a fragment of histone H4. Kayne P.S. et al., (1988) Cell 55:27-39; Kharchenho E.P., et al., (1987) Biull. Eksp. Biol. Med. 103.(4): 418-420. The references, however, do not disclose polypeptides within the scope of the present invention and do not disclose any of the biological properties of the polypeptides of present invention.

### SUMMARY OF THE INVENTION

The present invention provides a newly isolated biochemically pure polypeptide (or polypeptides) (i) having a stimulatory effect on osteoblastic cells, and (ii) which promotes in vivo bone formation. The present invention provides a biochemically pure polypeptide (OGP) of the formula:
Ala-Leu-Lys-Arg-Gln-Gly-Arg-Thr-Leu-Tyr-Gly-Phe-Gly-Gly
or a homolog, isoform or variant thereof which homolog, isoform or variant has a stimulatory effect on osteoblastic cells and in vivo bone formation, a molecular weight in the range of 500 to 2600 and an amino acid sequence which is at least 40% conserved in relation to OGP. This polypeptide was identified from regenerating bone marrow. The invention also provides a method of isolating the polypeptide from regenerating bone marrow. The polypeptides of the invention are useful to increase bone formation. The polypeptides of the present invention are also useful in pharmaceutical compositions, as screening tools and in the prevention, prophylaxis, therapy and treatment of diseases involving bone defects, bone loss and decreased bone formation, or other conditions which would benefit from increased bone formation.

### DESCRIPTION OF THE DRAWINGS

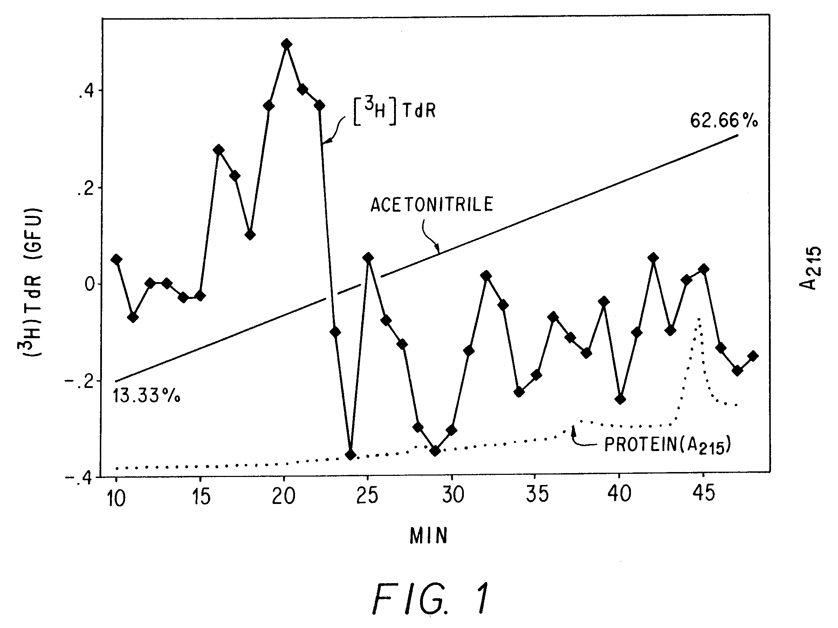

Figure 1 shows C1/C8 reverse-phase chromatography of GFA obtained by cation-exchange chromatography. The solid line represents DNA synthesis rates in ROS 17/2 cell cultures. Data are mean counts per minute from triplicate cultures. All fractions in the peak region as well as all other data points had an SD<10% of the mean. Dotted line represents the protein content.

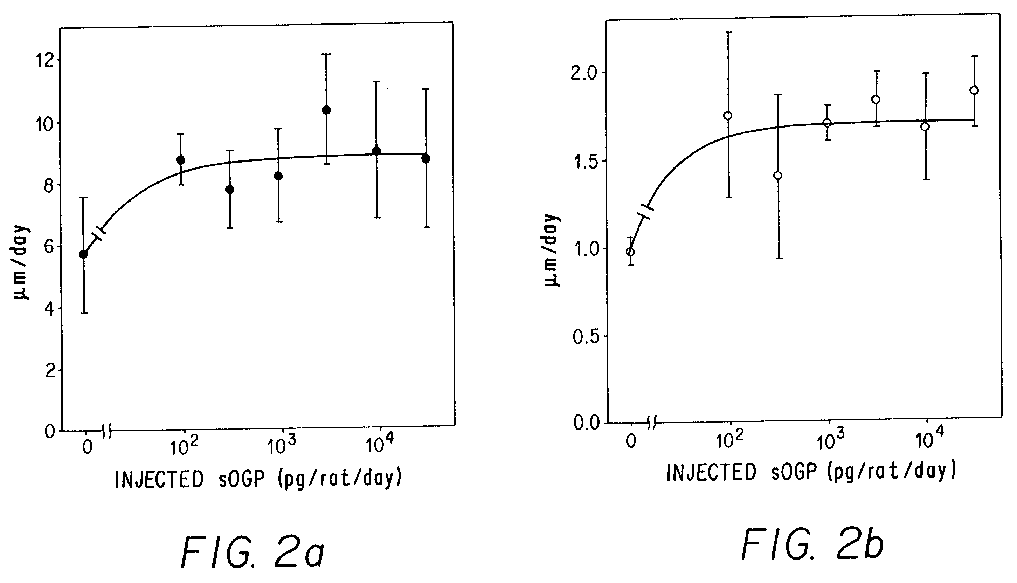

Figure 2 shows the effect of sOGP on mineral appositional rate (MAR) in cortico-endosteal (a) and metaphyseal trabecular (b) surfaces of rat tibia. Data are mean±SD of measurements in 4 (100 and 300 pg groups) or 5 (other groups) rats, one tibia per animal.

### DETAILED DESCRIPTION OF THE INVENTION

Osteogenic growth polypeptide ("OGP") is a biochemically pure polypeptide identified from regenerating bone marrow and having a stimulatory effect on osteoblastic cells and in vivo bone formation. OGP, as used herein, is defined to include native polypeptides, synthetic polypeptides, all homologs, isoforms or genetic variants, and all other variants. The molecular weight of OGP is in the range of about 500 to 2600, preferentially of about 1000 to 1600, and more preferentially of about 1525.

OGP was purified to three different stages, from regenerating (or healing) bone marrow conditioned media (HBMCM), Stages 1 and 2A having partial purity, and Stage 2B having apparent homogenity.

The invention identifies as an effective stimulator of bone formation a 14-residue polypeptide. The native OGP was isolated and purified to homogeneity from culture media conditioned by osteogenic tissue obtained from rat tibiae during post-ablation marrow regeneration (HBMCM). The purification procedure consisted of chromatography and selection of chromatographic fractions showing biological activity, including size exclusion, heparin-Sepharose, ion-exchange and reverse-phase chromatography.

Following demonstration of its mitogenic activity, the purified polypeptide OGP was subjected to automated Edman degradation for amino acid sequence analysis, A synthetic polypeptide of identical sequence was prepared by solid-phase peptide synthesis. The synthetic polypeptide OGP (sOGP) was tested and found to have a stimulatory effect on osteoblastic cells. When injected intravenously daily to adult rats of about 200-250 g, sOGP promoted bone formation (measured as mineral apposition rate) at doses of from about 1 pg /rat/day to about 1 µg/rat/day.

The data suggests that OGP is a single polypeptide of identified sequence, however, the possibility of homologs, isoforms or genetic variants of OGP exists either within or outside the cellular environment. This invention encompasses all such homologs, isoforms or genetic variants of OGP, provided each has an effect on osteoblastic cells and on in vivo bone formation. Polypeptides that are homologs of OGP specifically include those having an amino acid sequence which is at least about 40% conserved in relation to the amino acid sequence set forth in Table A, preferentially at least about 60% conserved, and more preferentially at least about 75% conserved.

It will be understood by one or ordinary skill in the art that other variants of OGP are included within the scope of the present invention. This particularly includes any variants that differ from the isolated or synthesized OGP only by conservative amino acid substitution. Many such conservative amino acid substitutions are set forth as sets in Taylor, W.R., J. Mol. Biol. 188, 233 (1986). OGP, or fragments thereof, in this application includes any such variations in the amino acid sequence, whether by conservative amino acid substitution, deletion, or other processes, provided that the polypeptide after purification shows a stimulatory effect on osteoblastic cells and on in vivo bone formation. The fragments of OGP may be small peptides with sequences of as little as 6 or more amino acids, said sequences being those disclosed in Table A.

The amino acid sequence of OGP is as follows:

### Table A

Ala-Leu-Lys-Arg-Gln-Gly-Arg-Thr-Leu-Tyr-Gly-Phe-Gly-Gly

It will be readily apparent to one of ordinary skill in the art that a great deal of use can be made of the amino acid sequence of OGP. For example, oligonucleotide probes can be constructed from the amino acid sequence and employed to screen for the cDNA clones encoding OGP. These clones containing OGP cDNA(s) can be used to transcribe mRNA which can then be translated and expressed. This work with OGP can be used to produce large quantities of OGP by genetic engineering or to study the genetics of OGP to learn its cellular role in bone formation.

Additionally, synthetic polypeptides can be made in order to improve upon the pharmacological properties of OGP. These synthetic peptides can be made by the technique of solid-phase peptide synthesis developed by Merrifield ("Solid-Phase Peptide Synthesis", Advances in Enzymology, 32:221-296, 1969); G. Barnay & R.B. Merrifield "Solid-Phase Peptide Synthesis", The Peptides, Vol. 2, ed. E. Gross & J. Merenhole (1980). This method is based on the strategy of having the carboxyl terminus of the peptide linked covalently to a solid support. The desired peptide sequence is prepared by stepwise coupling of single amino acids to a peptide chain growing from the carboxyl toward the amino terminus. Coupling is typically achieved by activation of the carboxyl group of the amino acid being attached to the resin which may have other potentially reactive groups blocked. Following addition of amino acid to the growing polypeptide chain, and prior to further chain elongation, a protecting group is typically removed. Because each amino acid is coupled by nearly the same series of reactions, the need for elaborate strategies in the synthesis is minimized. Solubility is not a major issue during synthesis, because the peptide is linked to a solid support. This method is rapid and it can be utilized simply. It is very convenient for the synthesis of multiple analogs with amino-terminal substitutions, because a single synthesis can be branched in multiple directions near the amino terminus, thereby creating many analogs varying only in the amino terminal region.

Additionally, the amino acid sequence can be used to make polypeptides which can be used as a screen or tool for the identification of non-peptidal molecules which show a stimulatory effect on osteoblastic cells and in vivo bone formation.

The polypeptides of the present invention also find utility for the stimulation of bone formation, in instances of osteoporosis (or osteopenia of any etiology), fracture repair, healing of osseous defects or wounds, intraosseous implants, and bone supplementation, or other conditions requiring increased bone formation.

The polypeptides of the present invention can be included in pharmaceutical compositions for the treatment or prevention of diseases involving a reduction in bone formation as well as the other conditions discussed above.

The magnitude of a prophylactic or therapeutic dose of a polypeptide of this invention will, of course, vary with the group of patients (age, sex, etc.), the nature or the severity of the condition to be treated and with the particular polypeptide of this invention and its route of administration. In general, the daily dose range for bone formation enhancing use lies within the range of from about 4 pg to about 5 µg per kg body weight of a mammal.

Any suitable route of administration may be employed for providing a mammal, especially a human with an effective dosage of a polypeptide of this invention. For example, oral, rectal, topical, parenteral, ocular, nasal, sublingual, buccal, intravenous and the like may be employed. Dosage forms include tablets, troches, dispersions, suspensions, solutions, capsules, creams, ointments, aerosols and the like. Said dosage forms also include implanted slow releasing devices specifically designed for this purpose or other forms of implants modified to additionally act in this fashion.

The pharmaceutical compositions of the present invention comprise a polypeptide of this invention as an active ingredient or a pharmaceutically acceptable salt thereof, and may also contain a pharmaceutically acceptable carrier and optionally other therapeutic ingredients. The term "pharmaceutically acceptable salts" refers to salts prepared from pharmaceutically acceptable non-toxic bases including inorganic bases and organic bases. The compositions include compositions suitable for oral, rectal, ophthalmic, pulmonary, nasal, sublingual, dermal, topical or parenteral (including subcutaneous, submucosol, intramuscular, intravenous and intra-arterial) administration, although the most suitable route in any given case will depend on the nature and severity of the conditions being treated and on the nature of the active ingredient. They may be conveniently presented in unit dosage form and prepared by any of the methods well-known in the art of pharmacy.

For administration by inhalation, the polypeptides of the present invention are conveniently delivered in the form of an aerosol spray presentation from pressurized packs or a nebuliser, or a powder which may be formulated as a cartridge from which the powder composition may be inhaled with the aid of a suitable device. The preferred delivery system for inhalation in a metered dose inhalation (MDI) aerosol, which may be formulated as a suspension or solution in fluorocarbon propellants.

Suitable topical formulations include transdermal devices, aerosols, creams, ointments, lotions, dusting powder, and the like.

In practical use, a polypeptide of this invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous and intra-arterial). In preparing the compositions for oral dosage form, any of the usual pharmaceutical media may be employed, such as, for example, water glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, capsules and tablets. Because of their ease of administration, tablets and capsules represent the most advantageous oral dosage unit form, in which case solid pharmaceutical carriers are obviously employed. If desired, tablets may be sugar coated or enteric coated by standard techniques.

In addition to the common dosage forms set out above, the polypeptides of this invention may also be administered by controlled release means and/or delivery devices.

Pharmaceutical compositions of the present invention suitable for oral administration may be presented as discrete units such as capsules, cachets or tablets each containing a predetermined amount of the active ingredient, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent.

The following examples are offered by way of illustration and not by way of limitation.

### EXAMPLE 1 (STAGE 1 PURIFICATION)

### MATERIALS

Reagents for assay of alkaline phosphatase activity, collagenase type I, trypsin, soybean trypsin inhibitor-agarose (STI), BGJ medium (Fitton-Jackson modification), vitamin C, bovine pancreas insulin, and human serum albumin, were purchased from Sigma Chemical Co. (St. Louis, MO). F-10 (Ham) medium (nutrient mixture), fetal calf serum (FCS), Dulbecco's PBS, and penicillin-streptomycin solution were obtained from Gibco (Chagrin Falls, OH). [methyl-³H]Thymidine ([³H]TdR) (5mCi/mmol) was from Nuclear Research Center (Negev, Israel). Sephadex G-25, Sephadex G-75, and heparin-Sepharose CL-6B were purchased from Pharmacia (Uppsala, Sweden). Millipore membranes were from Schleicher & Schuell (Dassel, West Germany). Platelet-derived growth factor (PDGF) was purchased from Biomedical Technologies (Stroughton, MA) and human recombinant interleukin I-a(IL1) from cistrone (Pine Brook, NJ). All other chemicals were of analytical grade and purchased from Merck AG (Darmstadt, West Germany). Tissue culture dishes were obtained from Nunc (Roskilde, Denmark). Rabbit polyclonal antiserum against PDGF was provided by Dr. C. H. Heldin (Uppsala University, Sweden).

### METHODS

### Preparation of Healing Marrow Conditioned Medium (HBMCM)

Tibial marrow was ablated from one limb of each of 400 g male rats of the Hebrew University (Sabra, Israel) strain as described previously. Bab, I. et al., (1985) Calcif Tissue Int 37:551. Briefly, a hole, 2 mm in diameter, was drilled in the shaft at a level below the proximal growth plate. Tissue was then removed from the marrow space with a polyethylene cannula inserted through the hole and attached to a high powered suction apparatus. The treated bones were dissected after 10 days and the shafts split longitudinally to expose the marrow space. Healing tissue was then removed from the endosteal aspect of the cortex, washed with copious amounts of serum-free F-10 medium supplemented with 1% (vol/vol) penicillin-streptomycin and incubated for 24 hour in the same medium (tissue from one limb/1 ml medium) at 37°C in 5% CO₂-air. The medium was then collected and centrifuged for 30 min at 25,000 x g, and the supernatant was filtered through a 0.45-mm pore-size Millipore membrane. The preparation was designated crude HBMCM; its protein content was 3-8 mg/ml. To remove cold thymidine, components of the tissue culture medium, and other low molecular weight contaminants, the crude HBMCM was subjected to gel filtration on a Sephadex G-25 column equilibrated with 5 mM ammonium-acetate. In standard experiments 6 or 35 mg protein were applied onto a PD-10 or 2.6 x 70 cm columns, respectively. Fractions were eluted with 5 mM ammonium-acetate and those containing protein in the void volume were pooled, lyophilized, and stored at -70°C. For further experiments samples were thawed and dissolved in PBS.

### Monitoring Growth Promoting Activity to Osteoblastic Cells

Growth factor activity (GFA) was monitored by examining effects on DNA synthesis in a culture of osteoblastic rat osteosarcoma cells (ROS 17/2). Stock cultures of ROS-17/2 cells were maintained in F-10 medium containing 10% FCS. To study the mitogenic effect of different preparations, confluent cultures were trypsinized, and 2 x 10⁴ cells seeded in 2 cm² culture wells (16 mm multiwell dishes) in F-10 medium and incubated at 37°C in CO₂-air. During the first 6 h the medium was supplemented with 2% FCS to enhance cell anchorage. This was followed by 18 hour incubation in serum-free medium that contained the test preparation added as protein solution in PBS. To determine DNA synthesis rates, cultures were pulsed with [³H]TdR, 1.5 mCi/well, for the last 2 hour of the incubation period. The pulse was terminated by washing twice with ice cold 10% (wt/vol) trichloroacetic acid and with ethanol-ether (3:1, vol/vol). After the cell layer had dried, the trichloroacetic acid-insoluble material was dissolved in 0.2 M NaOH and its total radioactivity estimated by liquid scintillation spectrometry. Data were expressed as growth factor units (GFU). Since the growth of ROS cells is serum dependent, 1 U was defined as half the effect of 10% FCS in a given experiment. Cell number was determined in sister cultures exposed to test preparations for 48 hour. This was done after trypsinization using a fixed volume hemocytometer. The data were expressed as the number of cells per culture well.

### Partial Purification of GFA from HBMCM

### Effect of Heat

One milliliter aliquots of HBMCM containing 0.5-1.0 mg protein were left at room temperature or heated to 56°C for 30 or 60 min. Similar samples were also tested for stability of the GFA to boiling for 10 min. Since the GFA was found to be stable to the boiling procedure (Table 1) a boiling step was used for the removal of extraneous protein by denaturation and centrifugation for 45 min at 25,000 x g.

### Affinity Chromatography

A heparin-Sepharose column (0.9 x 25 cm bed volume) was prepared according to manufacturer's instructions, packed with PBS, and pumped at room temperature at a flow rate of 0.6 ml/min. Preliminary experiments using heparin-Sepharose batchwise indicated that at equilibrium with 0.15 M NaCl (PBS) the GFA was left unbound to the insoluble substrate. Thus, a 2-ml sample containing 30 mg boiled HBMCM in PBS was loaded onto the column and eluted by washing the bed with 24 ml PBS. The eluate was dialyzed for 24 h against 5 mM ammonium acetate, assessed for protein, and lyophilized.

### Gel Filtration

To further purify the HBMCM-derived factor(s), 0.5-6.0 mg/ml of protein that was recovered from the heparin-Sepharose column were dissolved in 1 ml of 5 mM ammonium acetate and applied onto a 1.2 x 54 cm Sephadex G-75 column equilibrated with the same solution. The protein samples were eluted at room temperature using 5 mM ammonium acetate at a flow rate of 0.65 ml/min. Fractions of 1.3 ml were assessed for protein and lyophilized.

### Tryptic Digestion

To confirm the proteinaceous nature of the HBMCM-derived factor, 1-ml aliquots of HBMCM after the heparin-Sepharose step were incubated with trypsin (trypsin/HBMCM ratio 1:20 wt/wt) at 25°C. The reaction was stopped after 30 min by applying the mixture to a column of STI (0.4 x 1 cm). Controls consisted of samples treated similarly but without trypsin in the reaction mixture.

### Other Cell and Organ Cultures

To study the specificity of the HBMCM-derived factor to osteogenic cells, active preparations were further tested for their mitogenic effect on osteoblastic and nonosteoblastic fetal rat calvarial cells (FRC cells), and nonosteogenic rat osteosarcoma cells (ROS 25/1). Cultures were uniformly kept at 37°C in CO₂-air. In all experiments test preparations were added to cultures as protein solutions in PBS.

### ROS cells

ROS 25/1 cells were cultured and tested using a protocol similar to that reported above for ROS 17/2 cells.

### FRC cells

Cells obtained from parietal bones of 21-day rat fetuses were used in primary cultures. Five cell populations were separated by sequential digestion with collagenase and trypsin according to the method described by Luben et al., (1976) Endrocrinology 99:526. Cells from populations 1-2 and 3-5 were pooled and designated nonosteoblastic and osteoblastic, respectively. The cells were seeded in 16-mm multiwell dishes, 3 x 10⁴ cells per well, and allowed to grow for 24 h in F-10 medium supplemented with 10% FCS. The medium was then replaced by one with 1% FCS, and after 24 h the test preparation and [³H]TdR, 1.5 mCi/well, were added. Incorporation of [³H]TdR into DNA and cell number were assessed as described above after an additional 24-hour period.

### Fetal Mouse Long Bone

This was carried out as described by Soskolne et al., (1986) bone 7:41. Briefly, radii and ulnae were removed from 16-day fetuses and dissected free of muscle and soft tissue. They were then cultured in a chemically defined medium (BGJ, Fitton-Jackson modification) supplemented with 150 mg/ml vitamin C and 4 mg/ml human serum albumin. Phosphate concentration was adjusted to 1 mM. Total and diaphyseal lengths of individual bone rudiments were measured at the beginning of the culture period and after 48 hour directly under a dissecting microscope using transmitted light. Elongation, either total or diaphyseal, was calculated as the difference between these measurements, and the results were expressed as the ratio between bones treated with growth factors and controls grown in a chemically defined medium only (T/C ratio).

### Alkaline Phosphatase Activity

For this assay the medium was removed from cultures of ROS 17/2 cells, and the cells were washed with PBS, scraped into distilled water, and sonicated. Enzyme activity was assayed with p-nitrophenyl phosphate as a substrate as described by Ashton et al., (1984) Calcif Tissue Int 36:83. The results were expressed as micromoles of p-nitrophenol released per min/10⁶ cells that were counted in sister cultures.

### Protein Content

Protein was determined according to the method of Bradford, (1976) Anal. Biochem. 72:248.

### Assessment of IL1 Activity

IL1 activity was estimated using a thymocyte proliferation assay as described by Barak et al., (1986) J. Biol. Response Modifies 5:362.

### Assay for PDGF

Anti-PDGF antibody neutralization experiments were carried out with polyclonal antiserum prepared in rabbits against PDGF. PDGF or HBMCM-derived preparations were added to ROS 17/2 cells in the presence or absence of antibody and examined for effects on [³H]TdR incorporation.

### RESULTS

The effect of crude HBMCM on the number and alkaline phosphatase activity of ROS 17/2 cells is established. At the highest dilution of crude HBMCM (1:200) there was a 75% decrease in cell number and more than 2-fold increase in enzyme activity. At lower dilutions, 1:100-1:10, the number of cells was approximately 2-fold higher compared to untreated cultures, and alkaline phosphatase activity showed a dose-dependent reduction.

When crude HBMCM was subjected to gel filtration on Sephadex G-25 column, approximately 80% of the added protein was recovered in fractions comprising the void volume. These fractions contained nearly all of the (94%) mitogenic activity eluted from the column.

### Partial Purification of GFA From HBMCM

### Effect of heat on GFA.

The effect of heat on HBMCM-derived GFA is summarized in Table 1. GFU are growth factor units. 1 U is defined as half the effect of 10% FCS in the same experiment.

**TABLE 1**

| Effect of heat on HBMCM mitogenic activity ([³H]TdR incorporation into DNA) in serum-free ROS 17/2 cell culture | | |
|---|---|---|
| Factor^{a} | Counts per min^{b} | GFU^{b} |
| HBMCM (60 min, RT) | 2038 ± 304 | 0.29 ± 0.05 |
| HBMCM (30 min, 56 C) | 2792 ± 125 | 0.59 ± 0.07 |
| HBMCM (60 min, 60 C) | 3213 ± 89 | 0.82 ± 0.05 |
| HBMCM (10 min, boiling) | 5820 ± 243 | 2.82 ± 0.18 |
| Serum-free control | 1676 ± 130 | |
| Serum (10%) control | 5385 + 350 | |
| RT, Room temperature. | | |

| | | |
|---|---|---|
| ^{a}Factor concentration was 3.6 mg/ml. | | |
| ^{b}Mean ± SE of four replicate culture wells. | | |

With the elevation of either temperature or exposure time, there was an increase in the stimulatory effect of HBMCM on [³H]TdR incorporation into DNA of ROS 17.2 cells. In particular, there was a marked increase in mitogenic activity after 10 min boiling; the mitogenic effect of the supernatant obtained by boiling and centrifugation was similar to that of FCS. Half the protein could be removed by boiling and centrifugation with 8-fold increase in specific activity (Table 2). The dose-response relationship of the boiled preparation in the ROS 17/2 cell assay indicated significant GFA at 0.5-5 mg/well with a decline at higher doses. Activity of the boiled preparation was also noted when 0.5 mg was added to cultures of a pool of FRC cell populations 3-5 (osteoblastic population).

**TABLE 2**

| Partial purification of HBMCM-derived growth factor by boiling and affinity chromatography on heparin-Sepharose | | | | |
|---|---|---|---|---|
| Step of Purification | Protein (mg)^{a} | Recovered Activity (GFU) | Specific Activity (GFU/mg) | Purification |
| Sephadex G-25 | 5 | 495 | 99 | 1 |
| Boiling | 2.5 | 2,230 | 822 | 8 |
| Heparin-sepharose | 0.5 | 30,000 | 60,000 | 606 |

| | | | | |
|---|---|---|---|---|
| ^{a}From 1 ml crude HBMCM. | | | | |

### Affinity Chromatography.

When boiled HBMCM was loaded onto a heparin-Sepharose column, 20% of the applied protein was eluted by PBS, with the remaining protein left bound. The mitogenic activity recovered in the fraction eluted by PBS represented a 13 fold increase in specific activity (Table 2). The enhancement of GFA after the heparin-Sepharose step is also shown by the dose-response relationship where the effect on ROS 17/2 cells was apparent at 50 ng/well. The peak stimulation of DNA synthesis rate was found at 0.5 µg/well with a decline thereafter. The preparation obtained using heparin-Sepharose had also a considerable mitogenic effect on osteoblastic FRC cells. When material recovered from the heparin-Sepharose column was subjected to tryptic digestion, there was more than 95% inhibition of HBMCM derived GFA (Table 3).

**TABLE 3**

| Effect of tryptic digestion on stimulation of ROS 17/2 cell DNA synthesis by HBMCM chromatographed on heparin-Sepharose | | |
|---|---|---|
| Preparation added^{a} | Counts per min^{b} | GFU^{b} |
| HBMCM^{c} | 7245 ± 357 | 3.59 ± 0.28 |
| HBMCM + STI^{d} | 6309 ± 683 | 2.89 ± 0.52 |
| HBMCM + trypsin + STI | 1502 ± 69 | 0.13 ± 0.05 |
| Serum-free control | 1330 ± 75 | |
| Serum (10%) control | 3998 + 68 | |

| | | |
|---|---|---|
| ^{a}In absence of serum unless otherwise specified. | | |
| ^{b}Mean ± SE of four replicate culture wells. | | |
| ^{c}Two micrograms per culture of HBMCM preparation obtained by heparin-Sepharose chromatography. | | |
| ^{d}Two micrograms per culture of HBMCM preparation obtained after heparin-Sepharose and soybean trypsin inhibitor (STI)-agarose chromatograph. | | |

### Gel Filtration on Sephadex G75

The elution profile of GFA from the Sephadex G75 column visualized by enhancement of [³H]TdR incorporation into DNA of ROS 17/2 cells was established. Most of the protein and some mitogenic activity eluted close to the void volume of the column. Three major peaks of activity eluted in fractions 19 to 38. Based on the elution positions of mol wt markers, the mol wt estimates of the three peaks were 35,000, 19,000 and less than 10,000.

### Effect of HBMCM-derived Preparations In Other Cell and Organ Cultures

Nonosteoblastic ROS 25/1 cells had been obtained from the same tumor as ROS 17/2 cells but unlike the latter they do not express the osteoblastic phenotype. HBMCM-derived preparations, in particular those obtained after the heparin-Sepharose step, elicited some mitogenic response in the ROS 25/1 cell culture at concentrations similar to those stimulatory to ROS 17/2 cells. However, the magnitude of the ROS 25/1 cell response was considerably smaller compared to that of ROS 17/2 cells. In addition, the HBMCM-derived preparations did not have an apparent effect on DNA synthesis rates of nonosteoblastic FRC cells (populations 1-2).

When boiled HBMCM was added to an organ culture of fetal radii and ulnae there was a marked dose-dependent enhancement of growth expressed by increases in both diaphyseal and total elongation. The peak effect was found at 8 µg/ml protein concentration. At this concentration the increase of diaphyseal and total lengths was approximately 200% and 250% over growth factor-free controls, respectively. The difference in the magnitude of elongation between the diaphysis and whole rudiment resulted from enhanced growth of the cartilaginous epiphyseal ends. The peak effect of boiled HBMCM was nearly twice that of a positive insulin control.

### Thymocyte Proliferation Assay for IL1 Activity

Table 4 shows that unlike IL1 preparations, in medium containing PHA, neither boiled HBMCM nor the derivative obtained after the heparin-Sepharose step stimulated [³H]TdR incorporation into thymocyte DNA, suggesting that the HBMCM-derived growth factor does not resemble IL1.

**TABLE 4**

| Effect of IL1 and HBMCM preparations on incorporation [³H]TdR into DNA of isolated murine thymocytes | | |
|---|---|---|
| Factor^{a} | Concentration | Counts per min^{b} |
| Boiled HBMCM | 0.4 µg/ml | 1770 ± 13 |
| | 4.0 µg/ml | 1613 ± 11 |
| HS-HBMCM^{c} | 0.7 µg/ml | 2667 ± 5 |
| | 1.1 µg/ml | 2290 ± 17 |
| IL1^{d} | 5.0 U/ml | 7634 ± 35 |
| IL1^{e} | 5.0 U/ml | 6532 ± 30 |
| PHA | 10.0 µg/ml | 2590 ± 20 |
| -PHA | | 400 + 46 |

| | | |
|---|---|---|
| ^{a} HBMCM and IL1 preparations were tested in the presence of 10.0 mg.ml PHA. | | |
| ^{b} Mean ± SE of eight culture microwells. | | |
| ^{c} HBMCM after heparin-Sepharose step. | | |
| ^{d} From human monocytes. | | |
| ^{e} Human recombinant. | | |

### PDGF Content

Addition of polyclonal anti-PDGF antibodies to ROS 17/2 cells inhibited PDGF-stimulated replication but did not reduce the mitogenic effect produced by intermediate doses of boiled HBMCM and the preparation obtained after the heparin-Sepharose step. Under these conditions the presence of a significant amount of PDGF in the HBMCM-derived preparations should have resulted in decreased enhancement of ROS cell proliferation when tested with the antiserum.

### EXAMPLE 2 (STAGE 2A PURIFICATION)

### MATERIALS AND METHODS

### Materials

F-10 (HAM) medium (nutrient mixture), fetal calf serum (FCS), Dulbecco's phosphate buffered saline (PBS) and penicillin-streptomycin solution were obtained from Gibco (Chagrin Falls, OH). [methyl-³H]thymidine ([³H]TdR) (5 µCi/mmol) was from Nuclear Research Center (Negev, Israel). Heparin-Sepharose CL-6B was purchased from Pharmacia (Uppsala, Sweden). Reagents for alkaline phosphatase assay were obtained from Sigma (St. Louis, MO) and chemicals for SDS-PAGE from Bio-Rad (Richmond, CA). Transforming growth factors beta 1 (TGFβ1) and beta 2 (TGFβ2) were obtained from R&D Systems (Minneapolis, MN) Insulin-like growth factor I (IGF-I) was obtained from Merck, Sharp and Dohme Research Laboratories, Rahway, NJ. All other chemicals were of analytical grade and purchased from Merck AG (Darmstadt, W. Germany). Rat osteosarcoma (ROS) cells were obtained from Drs. G.A. and S.B. Rodan (Merck, Sharp and Dohme Research Laboratories, West Point, PA). Tissue culture dishes were obtained from Nunc (Roskilde, Denmark).

### Preparation of Healing Marrow

### Conditioned Medium (HBMCM)

Conditioned medium was prepared from healing marrow as described previously by Bab, I. et al (1988) Endocrinology 123:345. Briefly, tissue was separated from the marrow space of rat tibiae 10 days after ablation and incubated for 24 hours in serum-free F-10 medium supplemented with 1% (vol./vol.) penicillin-streptomycin at 37°C in 5% CO₂-air. The medium was then collected, boiled for 10 minutes, centrifuged for 30 minutes at 25,000 xg and filtered through 0.45-µm pore size Millipore membrane.

### Cell Cultures

Growth factor activity (GFA) was monitored by examining effects on DNA synthesis in a culture of osteogenic ROS 17/2 cells. In short, HBMCM-derived preparations and other growth factors were added to 2 cm² culture wells (16 mm multiwell dishes) containing the ROS cells in serum-free F-10 medium. After 22 hours, the cultures were pulsed with [³H]TdR, 2 µCi/ml. Two hours later, the total radioactivity of trichloroacetic acid insoluble material was determined by liquid scintillation spectrometry and the data expressed as percent over untreated cultures or growth factor units (GFU). One unit was defined as half the effect of 10% fetal bovine serum in a given experiment. To assess specificity of the HBMCM derivatives to osteogenic cells, some preparations were tested in cultures of non-osteogenic ROS 25/1 cells using a protocol similar to that described above for the ROS 17/2 cells.

### Affinity Chromatography

A heparin-Sepharose column (0.9x25 cm bed volume) was prepared according to the manufacturer's instructions, packed with PBS and pumped at room temperature at a flow rate of 0.5 ml/minutes. A 2 ml sample containing 28 mg boiled HBMCM in PBS was loaded onto the column and eluted in two steps. First, the heparin-Sepharose bed was washed isocratically with PBS for 200 minutes. Then a two-stage linear gradient of 0.15-1.35 M NaCl in phosphate buffer, pH 7.2, was pumped through the column. The gradient rate was 0.015 M/minute and 0.005 M/minute during stages I and II, respectively. Two ml fractions were collected and dialyzed for 24 hours against 5 mM ammonium-acetate, assessed for protein and lyophilized.

### Inactivation Experiments

Samples of heparin-Sepharose peak activity fractions containing approximately 10 GFU were dissolved in water and reacted for 90 minutes at 37°C with (a) 5 mM dithiothreitol (DTT); (b) 0.1 M HCl and (c) PBS control. The reactions were terminated by 5 hours dialysis against 5 mM ammonium acetate in the cold.

### Gel Electrophoresis

SDS-PAGE was performed in 1.5 mm thick 10-18% gradient gels according to Laemmli, Nature 227, 680 (1970).

### Alkaline Phosphatase Activity

For this assay ROS 17/2 cells were grown for 48 hours in F-10 medium supplemented with 2% FBS. During the last 24 hours, the cells were challenged with 10 µg/ml of either boiled or unboiled conditioned medium or 2 µg/ml of the heparin-Sepharose peak activity fractions. Then the medium was removed from the cultures and the cells washed with PBS, scraped into distilled water, and sonicated. Enzyme activity was assayed with p-nitrophenol phosphate as substrate. The results were expressed as micromoles of p-nitrophenyl released per min/10⁶ cells that were counted in sister cultures.

### Protein Content

Protein was determined according to the method of Bradford, Anal. Biochem. 72,248 (1976).

### RESULTS

The elution profile of GFA from the heparin-Sepharose column, visualized by enhancement of [³H]TdR incorporation into DNA of ROS 17/2 cells was obtained.

**TABLE 5**

| Heparin-Sepharose chromatography of HBMCM-derived growth factor activity: elution time¹ and maximal peak activity² | | | |
|---|---|---|---|
| Peak | Elution time (min) | Maximal activity (GFU) | Total protein (mg) |
| | | | |

| Stage I: isocratic elution with 0.15 M NaCl | | | |
|---|---|---|---|
| AII | 56.4 | 0.62±0.06 | 3.12 |
| AI | 130 | 1.55±0.05 | 0.61 |
| AIII | 184 | 1.02±0.04 | 0.13 |

| Stage II: elution with 0.15-1.35 NaCl gradient | | | |
|---|---|---|---|
| BI | 7.3 | 1.00±0.04 | 1.06 |
| BII | 39 | 1.59±0.02 | 0.80 |
| BIII | 130 | 0.84±0.04 | 0.06 |

| | | | |
|---|---|---|---|
| ¹ Elution time is presented separately from the onset of each stage. | | | |
| ² Fraction samples assayed for activity contained 2 mg/ml protein. Data are mean±SE of three replicate cultures per condition. | | | |

Three major peaks of activity, AI, AII and AIII, eluted when the column was washed isocratically with PBS (Table 5). Preparation A-I and particularly A-II were fairly stable to reduction with DTT and acidification with HCl (Table 6).

**TABLE 6**

| Stability of HBMCM-derived GFA peaks separated by heparin-Sepharose chromatagraphy | | | |
|---|---|---|---|
| Treatment | A-I | A-II | B-II |
| | % activity remaining | | |
| Boiling (HBMCM) | 100 | 100 | 100 |
| 5 mM DTT | 68 | 91 | 5 |
| 0.1 M HCl | 68 | 77 | 75 |

The resistance to reduction was further confirmed by gel electrophoresis where both A-I and A-II appeared similar on reduced and non-reduced gels. A-II contained a component migrating close to the gel's front and a few additional species of 60-75 KD. A-I consisted of several components at the 55-90 KD range. When the column was pumped with an NaCl concentration gradient. The respective GFA peaks, designated B-I, B-II and B-III, resolved at 0.3, 0.75 and 1.2 M salt, respectively. Preparation B-II was inactivated by reduction (Table 6) and on reduced gels showed several 55-80 KD bands. In addition, B-I and B-II contained 14 and 33 KD bands, respectively. The amount of protein recovered in preparations A-III and B-III was insufficient for their testing in the inactivation and electrophoresis experiments.

The conditioned medium prior to boiling failed to influence ROS cell alkaline phosphatase. Preparations A-II, B-II and B-III stimulated enzyme activity almost two-fold. The highest stimulatory effect, however, was seen when HBMCM was tested (300%).

Nonosteogenic ROS 25/1 cells had been obtained from the same tumor as ROS 17/2 cells but unlike the latter they do not express the osteogenic phenotype. Preparation A-II failed to stimulate incorporation of [³H]TdR into DNA of the ROS 25/1 cells. A-I showed some stimulation which was, however, only 40% its effect on ROS 17/2 cells. B-I had a similar effect on both cell types.

### DISCUSSION

During the osteogenic phase regenerating bone marrow produces growth-promoting activity to osteogenic cells. The present results show that the activity in HBMCM is divided among at least six independent activities separable by heparin-Sepharose affinity chromatography. Similarly, it has been demonstrated that growth factor activity obtained from demineralized bone matrix consists of several proteinous species also separable on heparin-Sepharose. Although multiple peaks in HBMCM could theoretically result from proteolytic degradation or aggregation of a factor with different "carrier" protein, this is unlikely since (a) inclusion of proteinase inhibitors during the medium conditioning and further processing did not alter the elution profile from heparin-Sepharose (data not shown) and (b) the properties of the separate GFA peaks with regard to stability and target cell action are clearly distinct.

### EXAMPLE 3 (STAGE 2B: PURIFICATION AND AMINO ACID SEQUENCING OF OGP)

### MATERIALS

F-10 (HAM) medium (nutrient mixture) and kanamycin sulfate were obtained from Grand Island Bological (Grand Island, NY). Fetal bovine serum (FBS) was from Hazelton/KC Biologicals (Leneta, KS). [methyl-³H]Thymidine ([³H]tdR) (6.7 Ci/mmol) was purchased from New England Nuclear (Boston, Ma.) Trans-epoxysuccinyl-leucyl-amido(4-guanidino)butane (E64), leupeptin and pepstatin were from Sigma Chemical Co. (St. Louis, MO). Heparin-Sepharose CL-6B and Sephadex G25 were obtained form Pharmacia (Uppsala, Sweden). Tissue culture dishes were the product of Costar (Cambridge, Ma).

### METHODS

### Partial purification of GFA from HBMCM:

HBMCM was prepared as described above (Example 1 and 2) and partially purified by boiling and Heparin-Sepharose chromatography using a modification of the protocol reported in Bab I., et. al (1988) Edocrinology 123:345. The HBMCM was boiled for 10 min and then centrifuged at 25,000 xg for 45 minutes in a cooled centrifuge. The supernatant was collected and supplemented with the following proteinase inhibitors: 25 µM E64, 25 µM leupeptin and 5 µM pepstatin. The same mixture of proteinase inhibitors was also added to preparations recovered from the subsequent heparin-Sepharose, gel filtration and ion-exchange steps (see below).

### Affinity Chromatography

A heparin-Sepharose column (1.6 x 24 cm bed volume) was prepared according to manufacturer's instructions, equilibrated with phosphate buffered saline (PBS) which was pumped at a flow rate of 0.5 ml/min at 4°C. Boiled HBMCM containing 100 mg protein was passed through the column. The column was further washed with 50 ml PBS. The recovered conditioned medium and PBS were then pooled and lyophilized. The heparin-Sepharose step was carried out repeatedly to accumulate partially processed GFA in amounts sufficient for further purification.

### Monitoring GFA in Osteogenic Cells

This was carried out in osteoblastic ROS 17.2 cells as described above (Example 1) with the exception that kanamycin-sulfate replaced the penicillin-streptomycin. The results were expressed as GFU or percent over PBS controls.

### Ion Exchange Chromatography

To remove salts, cold thymidine and other components of the tissue culture medium, the preparation recovered from the heparin-Sepharose column was dissolved in a small volume of water and passed over a prepacked Sephadex G25 column (PD-10). Ammonium-acetate (5mM) was used for column equilibration and elution. The void volume from multiple columns was collected and fractions showing GFA in the ROS 17.2 cell assay were pooled and lyophilized.

For ion-exchange chromatography 50 mM sodium acetate buffer (SAB), pH=5.0 was added to the lyophilized material, 1 ml/1.65 mg protein. The mixture was centrifuged at 10,000 xg for 15 min and the pellet, which contained about 85% of the protein in the mixture, was discarded. Samples of the supernatant containing 0.4-7.0 mg protein were chromatographed on a Mono-S HR 5/5 fast protein liquid chromatography (FPLC) cation exchange column (Pharmacia, Uppsala, Sweden) using Waters 650 Advanced Protein Purification System (Millipore Corporation, Milford, MA). The column was pumped at a flow rate of 1 ml/min in three stages: i. 3 min isocratically with the initial SAB; ii. 30 min linear gradient with 0-1.0 M NaCl in SAB and iii. 7 min with 1.0 M NaCl in SAB. One ml fractions were collected and samples containing approximately 30 ng protein were assayed for GFA. The results for each fraction were expressed as the percent of paired control samples consisting of the corresponding fractions obtained from the column during an identical run without loading the protein.

### Reverse-phase Chromatography

Fractions from multiple ion-exchange runs showing GFA were pooled and 3.2 ml with an estimated total protein content of 18 µg were loaded onto C1/C8 ProRPC HR 5/2 FPLC reverse-phase column (Pharmacia, Uppsala, Sweden). The column was eluted with 0-100% acetonitrile gradient containing a 0.1% trifluoroacetate (TFA) at a flow rate of 0.5 ml/min. The fractions, 0.5 ml each, were collected and dried in a Speedvac concentrator (Savant, Farmingdale, NY). Prior to this, 10 µl aliquots from each fraction were dried separately, redissolved in PBS and assayed for GFA.

### Amino acid sequencing:

Fractions recovered from the reverse-phase column that showed GFA were pooled and a sample containing ∼30 ng protein was subjected to automated Edman degradation for amino acid sequencing in a gas-phase protein sequencer Model 470A equipped with an on-line PTH analyser, Model 120A (Applied Biosystems, Inc., Foster City, CA).

### Protein Content:

Protein was determined as described above (Example 1).

### RESULTS

### Reverse-phase chromatography

The elution profile of reverse-phase chromatography is shown in fig. 1. Protein was recovered in two minor peaks (elution time 27 and 36 min) and one major peak (elution time 45 min). A major GFA peak was recovered after 19-22 min, an elution time corresponding to ∼27% acetonitrile.

The amino acid sequence of the protein recovered in the mitogenically active fractions (elution time 19-22 min, fig. 2) revealed a 14-residue peptide, MW 1523. There was no evidence for the presence of other contaminating peptides in this preparation. The sequence is shown in Table A.

### EXAMPLE 4

### (BIOLOGICAL ACTIVITY OF SYNTHETIC OGP)

### MATERIALS

t-Boc-Gly OCH₂-Pam resin, N-Boc protected amino acid derivatives, N,N dicyclohexylcarbodiimde (DDC), 1-hydroxybenzotriazole (HOBT), diispropylethylamine (DIEA), trifluoroacetic acid (TFA), N,N dimethylformamide (DMF) and dichloromethane (DCM) were obtained from Applied Biosystems Inc. (Foster City, CA). Hydrogen fluoride (HF) was purchased from Matheson (Secacus, NJ), p-Cresol from Aldrich Chemical Co. (Milwaukee, WI) and Sephdex G15F from Pharmacia (Uppsala, Sweden). F-10 (HAM) medium (nutrient mixture) and kanamycin sulfate were obtained form Grand Island Biological (Grand Island, NY) and fetal bovine serum (FBS) was from Polysciences Inc. (Warrington, PA). Male Sprague-Dawley rats weighing 240-260 g were from Taconic Farm, NY. Achromycin (tetracycline hydrochloride) was from Lederle (Pearl River, NY) and Terramycin (oxytetracycline) from Roerig-Pfizer (New York, NY). Ingredients of methyl-methacrylate embedding resin were the product of Fischer Scientific (Fair Lawn, NJ).

### METHODS

### Preparation of synthetic OGP (sOGP)

sOGP was synthesized by the solid phase method of Merrifield (1969) Adv. Enzymol. 32:221 using an Applied Biosystems Model 430A Automated Peptide Synthesizer (Applied Biosystems Inc., Foster City CA). The synthesis was carried out on 0.5 mmol t-Boc-Gly-OCH₂-Pam resin (1% cross linked, 0.78 mmol/g). The amino acid derivatives were protected on the α-amino function by t-butyloxycarbonyl (Boc) groups. Protection of the side chains was as follows: Arg (Tos), Lys (2-Cl-Z), Tyr (2-Br-Z) and Thr(O-Bzl). Coupling of the Boc protected derivative of Arg and Gln was by the DCC-HOBT method of Konig, W. and Geiger, R. (1970) Chem. Ber., 103:788. All other amino acid derivatives were coupled via the DCC-mediated preformed symmetrical and anhydride method of Hagemaier, H. and Frank H. (1972) Hoppe-Seyler's Z. Physiol. Chem. 353:1973. The coupling of each amino acid residue was repeated twice. Deprotection of the blocked amino-terminus was by treatment with 60% TFA in DCM. Side chains were deprotected and the peptide was cleaved from the resin (2.7 g resin-bound peptide) using the HF procedure where a mixture of 4 ml anisole and 36 ml liquid HF was used for 75 min at 0°C. The crude synthetic peptide was partially purified on a Sephadex G15F 3 x 35 cm column eluted with 50% (v/v) aqueous acetic acid. Further purification was accomplished on a Waters DeltaPrep 3000 High Pressure Liquid Chromatography instrument equipped with a PrePak 1000 module (Millipore Corporation, Milford, MA). The cartridge was pumped with 5-33.5% acetonitrile gradient containing 0.1% TFA at a flow rate of 100 ml/min.

### The Effect of sOGP on bone in vivo

sOGP in PBS solution was administered to rats daily via the tail vein, 100 µl/day/rat for 8 days. Control animals recieved PBS alone or a peptide having the reverse sequence sOGP. The rats were labeled twice with tetracycline by intramuscular injections of 6 mg achromycin and terramycin (in water) on days 2 and 8, respectively. The animals were sacrificed by cervical dislocation and the tibiae where separated and fixed in 70% ethanol. The specimens were then dehydrated, embedded in methyl-methacrylate and 10 µm undecalcified, unstained sections were subjected to fluorescent microscopy. Fluorescent images were recorded using a 480 nm fluoresecin filter in a Microphot epifluorescent microscope (Nikon, Japan) equipped with a SIT video camera (Dage-MTI, Michigan City, IN) connected to a Magiscan interactive image analyzer (Joyce-Loebl, Gateshead, UK). Measurements in the cortico-endosteal surface and the surface of the proximal metaphyseal trabeculae, were carried out on the analyzer screen at x550 magnification. Separation of double labels was determined as the mean of multiple measurements between the center of the lines in all double labeled zones in 10 microscopic fields. Mineral apposition rate (MAR) was expressed as micrometers per day of interlabel time space.

### RESULTS

Following 8 intravenous injections of sOGP both cortico-endosteal and metaphyseal trabecular surfaces of the tibia showed increase MAR (FIG. 2). The effective dose range was 0.1-30 ng/rat/day.

## Claims

1. A biochemically pure polypeptide (OGP) of the formula:
Ala-Leu-Lys-Arg-Gln-Gly-Arg-Thr-Leu-Tyr-Gly-Phe-Gly-Gly
or a homolog, isoform or variant thereof which homolog, isoform or variant has a stimulatory effect on osteoblastic cells and in vivo bone formation, a molecular weight in the range of 500 to 2600 and an amino acid sequence which is at least 40% conserved in relation to OGP.

2. A polypeptide according to claim 1 which is OGP.

3. A polypeptide according to claim 1 or 2 obtainable from regenerating bone marrow.

4. A polypeptide according to claim 1 which comprises a sequence of 6 or more amino acids.

5. A polypeptide according to claim 1 or 4 having a molecular weight of about 1000-1600.

6. A mixture comprising more than one polypeptide according to any one of the preceding claims.

7. A method for producing an osteogenic growth polypeptide or mixture thereof as defined in any one of the preceding claims which comprises isolating said polypeptide(s), free of biochemical debris, from regenerating bone marrow.

8. A method of identifying molecules having a stimulatory effect on osteoblastic cells and in vivo bone formation which comprises:
(a) contacting said molecule with a polypeptide or mixture thereof as defined in any one of claims 1 to 6; and
(b) measuring the effect on osteoblastic cells and in vivo bone formation relative to the effect in the absence of said molecule.

9. A pharmaceutical composition for increasing bone formation which comprises a therapeutically effective amount of a polypeptide or mixture thereof as defined in any one of claims 1 to 6 and a pharmaceutically acceptable carrier.

10. A polypeptide or mixture thereof as defined in any one of claims 1 to 6 for use in a method of treating the human or animal body for surgery or therapy.

## Patentansprüche

1. Biochemisch reines Polypeptid (OGP) der Formel:
Ala-Leu-Lys-Arg-Gln-Gly-Arg-Thr-Leu-Tyr-Gly-Phe-Gly-Gly
oder eine homologe Verbindung, isoforme Verbindung oder eine Variante davon, wobei die homologe Verbindung, isoforme Verbindung oder Variante eine stimulatorische Wirkung auf osteoblastische Zellen und auf die *in vivo*-Knochenbildung hat, ein Molekulargewicht im Bereich von 500 bis 2600 aufweist und eine Aminosäuresequenz hat, die zu mindestens 40 % in bezug auf OGP konserviert ist.

2. Polypeptid nach Anspruch 1, welches OGP ist.

3. Polypeptid nach Anspruch 1 oder 2, welches aus sich regenerierendem Knochenmark erhältlich ist.

4. Polypeptid nach Anspruch 1, welches eine Sequenz von 6 oder mehr Aminosäuren beinhaltet.

5. Polypeptid nach Anspruch 1 oder 4 mit einem Molekulargewicht von etwa 1000-1600.

6. Mischung, umfassend mehr als ein Polypeptid gemäß mindestens einem der vorhergehenden Ansprüche.

7. Verfahren zur Herstellung eines osteogenischen Wachstumsfaktor-Polypeptids oder einer Mischung davon, wie in einem beliebigen der vorstehenden Ansprüche definiert, welches das Isolieren des/der Polypeptide (s), frei von biochemischen Abfall- bzw. Trümmerprodukten, aus sich regenerierendem Knochenmark umfaßt.

8. Verfahren zum Nachweis von Molekülen mit einer stimulierenden Wirkung auf osteoblastische Zellen und die *in vivo*-Knochenbildung, welches folgenden umfaßt:
(a) Kontaktieren des Moleküls mit einem Polypeptid oder einer Mischung davon, wie in einem beliebigen der Ansprüche 1 bis 6 definiert; und
(b) Bestimmen der Wirkung auf osteoblastische Zellen und die *in vivo*-Knochenbildung im Vergleich zur in Abwesenheit dieses Moleküls vorliegenden Wirkung.

9. Pharmazeutische Zusammensetzung zur Steigerung der Knochenbildung, welche eine therapeutisch wirksame Menge eines Polypeptids oder einer Mischung davon, wie in einem beliebigen der Ansprüche 1 bis 6 definiert, und einen pharmazeutisch annehmbaren Träger umfaßt.

10. Polypeptid oder eine Mischung davon, wie in einem beliebigen der Ansprüche 1 bis 6 definiert, zur Verwendung bei der Behandlung des menschlichen oder tierischen Körpers zur Operation oder Therapie.

## Revendications

1. Polypeptide biochimiquement pur (OGP) de formule :
Ala-Leu-Lys-Arg-Gln-Gly-Arg-Thr-Leu-Tyr-Gly-Phe-Gly-Gly
ou un homologue, un isoforme ou variant de celui-ci, lequel homologue, isoforme ou variant a un effet stimulateur sur les cellules ostéoblastiques et la formation d'os *in vivo*, un poids moléculaire dans la gamme de 500 à 2600 et une séquence d'acides aminés qui présente un degré de conservation d'au moins 40% par rapport à OGP.

2. Polypeptide selon la revendication 1, qui est OGP.

3. Polypeptide selon la revendication 1 ou 2, pouvant être obtenu à partir de la moelle osseuse en voie de régénération.

4. Polypeptide selon la revendication 1, qui comprend une séquence de 6 acides aminés ou plus.

5. Polypeptide selon la revendication 1 ou 4 ayant un poids moléculaire de 1000 à 1600 environ.

6. Mélange comprenant plus d'un polypeptide selon l'une quelconque des revendications précédentes.

7. Procédé de production d'un polypeptide de croissance ostéogénique ou d'un mélange de polypeptides tels que définis dans l'une quelconque des revendications précédentes qui comprend l'isolement dudit ou desdits polypeptides, exempts de contaminants biochimiques, à partir de la moelle osseuse en voie de régénération.

8. Procédé d'identification de molécules ayant un effet stimulateur sur les cellules ostéoblastiques et la formation d'os *in vivo* qui comprend:
(a) la mise en contact de ladite molécule avec un polypeptide ou un mélange de polypeptides tels que définis dans l'une quelconque des revendications 1 à 6; et
(b) la mesure de l'effet sur les cellules ostéoblastiques et la formation d'os *in vivo* par rapport à l'effet observé en l'absence de ladite molécule.

9. Composition pharmaceutique pour augmenter la formation d'os qui comprend une quantité thérapeutiquement efficace d'un polypeptide ou d'un mélange de polypeptides tels que définis dans l'une quelconque des revendications 1 à 6 et un véhicule pharmaceutiquement acceptable.

10. Polypeptide ou mélange de polypeptides tels que définis dans l'une quelconque des revendications 1 à 6, pour utilisation dans un procédé de traitement du corps humain ou animal à des fins chirurgicales ou thérapeutiques.
